# EUROPEAN PATENT SPECIFICATION

(11) **EP 4 301 443 B1**
(45) Date of publication and mention of the grant of the patent: **27.08.2025**
(21) Application number: 22713036.6
(22) Date of filing: 07.03.2022
(51) Int. Cl.: A61M 16/06, A41D 13/11, A61M 16/00, A61M 16/08, A61M 16/10, A61M 16/20, A62B 18/02

(54) **SYSTEM AND METHOD FOR MAKING A SCREEN FOR C-PAP MEDICAL DEVICE OR PERSONAL PROTECTION DEVICE USING A SINGLE MOLD**
SYSTEM UND VERFAHREN ZUR HERSTELLUNG EINES SCHIRMS FÜR EIN C-PAP-MEDIZINISCHES GERÄT ODER EINE PERSÖNLICHE SCHUTZVORRICHTUNG UNTER VERWENDUNG EINER EINZIGEN FORM
SYSTÈME ET PROCÉDÉ DE FABRICATION D'UN ÉCRAN POUR UN DISPOSITIF MÉDICAL C-PAP OU UN DISPOSITIF DE PROTECTION INDIVIDUELLE EN UTILISANT UN MOULE UNIQUE

(30) Priority: 05.03.2021 IT 202100005267
(43) Date of publication of application: 10.01.2024
(73) Proprietor: Oldrati Guarnizioni Industriali S.p.A., 24060 Villongo (BG) (IT)
(72) Inventor: FRACASSI, Cristian, 25128 Brescia (IT); ROMAIOLI, Alessandro, 25010 San Zeno Naviglio (BS) (IT); SABAINI, Davide, 25016 Ghedi (IT)
(74) Representative: Autuori & Partners S.R.L.
(86) International application number: PCT/IB2022/051988
(87) International publication number: WO 2022/185291

(56) References cited:
- EP-A1- 0 304 641
- CN-A- 111 214 770
- CN-U- 210 672 174
- CN-U- 212 545 686
- US-A- 3 890 647
- US-A- 4 989 596
- US-A1- 2003 047 183

## Description

### Field of the Invention

The present invention generally relates to the technical field of protection systems and medical devices for mechanical ventilation, and it particularly has as object a system and method for making a screen to be used in a C-PAP medical device or in a personal protection device.

Another object of the present invention is a method for making the C-PAP medical device or the personal protection device including the making of the screen by the above system and method, as well as the C-PAP medical device and the personal protection device achievable with such method.

### Background of the invention

Depending on the types of orolaryngeal protection required, different types of protection devices are available, each of which must have protection characteristics defined by specific regulations according to use.

In particular, such devices can be classified in personal protective equipment (PPE) for the protection of an operator in a work environment potentially at risk due to the presence of harmful gases, dusts or harmful microparticles or in medical devices called C-PAP (*Continuous Positive Airway Pressure*) for the treatment of a patient with continuous positive pressure.

The well known abovementioned devices, in particular PPE and C-PAP devices, are made of thermoplastic polymeric material by molding the individual components. Considering a company that produces both categories of devices, which implies a series of molds and components for the PPE device and another distinct series of molds and components for the C-PAP medical device, i.e. two distinct design and manufacturing development processed and management of a double series of molds and a double series of components and respective warehouse and supply.

As well known, the mold is an expensive element and it is therefore appropriate to limit the number of molds to use, in order to limit the necessary investment and its immobilization in the warehouse when the mold is not in use.

A further cost is given by the mold change operations that necessarily require a downtime machine in production.

CN 111 214 770 A and US 2003/047183 A1 disclose fabrication of transparent screens for face shields by molding. US 4 989 596 A, CN 210 672 174 U, EP 0 304 641 A1, CN 212 545 686 U and US 3 890 647 A disclose face shields with transparent screens.

### Summary of the invention

Object of the present invention is to overcome, at least partially, the drawbacks illustrated above, by providing a system and a method for making a screen both for PPE devices and C-PAP medical devices being particularly cost-effective.

Another object of the present invention is to provide a system and a method for making a screen for both PPE devices and C-PAP medical devices being easy to implement.

Another object of the present invention is to provide a method for making PPE devices and C-PAP medical devices being particularly cost-effective and easy to implement.

Such objects, as well as others that will be clearer hereinafter, are fulfilled by a system and a method for making a screen both for PPE devices and C-PAP medical devices in accordance with claim 1 and claim 10 and 11.

The dependent claims define advantageous embodiments of the invention.

### Brief description of the drawings

Further features and advantages of the present invention will become more evident by reading the detailed description of some preferred but not exclusive embodiment of the invention, illustrated as a non-limiting example, with the help of the annexed drawings wherein:
**FIG. 1** is an exploded axonometric schematic view of an embodiment of the device 1 in configuration of a personal protection device;
**FIG. 2** is an axonometric schematic view of the device shown in FIG. 1 in assembled form;
**FIG. 3** is an exploded axonometric schematic view of an embodiment of the device **1** in configuration of the C-PAP medical device;
**FIG. 4** is an axonometric schematic view of the device shown in FIG. 3 in assembled form;
**FIGs. 5A, 5B** and **5C** are axonometric schematic figures of the embodiment of device **1** shown in FIG. 2 respectively in configuration for dental use, with camera for shooting surgeries, with a use/non-use signal light;
**FIGs. 6** and **7** are axonometric schematic views of the punch half-mold **70** for the molding of the screen **30** of device **1** shown in FIG. 3;
**FIGs. 8** and **9** are axonometric schematic views of the die half-mold **60** for the molding of the screen **30** of device **1** shown in FIG. 3;
**FIG. 10** shows an axonometric schematic view of the screen **30** for the C-PAP medical device produced by the punch half-mold **70** shown in FIGs. 6 and 7 and the die half-mold **60** shown in FIGs. 8 and 9;
**FIGs. 11** and **12** are axonometric schematic views of the punch half-mold **70** for the molding of the screen **30** of device **1** shown in FIG. 1;
**FIGs. 13** and **14** are axonometric schematic views of the die half-mold **60** for the molding of the screen **30** of device **1** shown in FIG. 1;
**FIG. 15** is an axonometric schematic view of the screen **30** for the personal protection device produced by the punch half-mold **70** shown in FIGs. 11 and 12 and the die half-mold **60** shown in FIGs. 13 and 14;
**FIG. 16** is a schematization of the embodiment of devices **1** shown in FIGs. 1 and 3.

### Detailed description of some preferred embodiments.

With reference to the above attached figures, it is here described a face-shield device **1** susceptible to be worn by a user in order to cover eyes, nose and mouth.

As better explained hereinafter, depending on configuration, the device **1** may be a personal protection device (PPE) or a device for the C-PAP mechanical ventilation in accordance with the ISO 17510 and ISO 5356 standard regulations so that when interacting with the user a constant positive pressure is created, allowing the pulmonary alveoli to never get completely empty.

In this last case the C-PAP device may comprise an inlet connectable to an oxygen supply line and an outlet fluidically connectable to a valve suitable to create pressure losses along the line,
Therefore, the device **1** comprising filter elements may be a personal protection device while the device **1** comprising an inlet connectable to an oxygen supply line and an outlet fluidically connectable to a valve suitable to create pressure losses is a device for the C-PAP mechanical ventilation.

The device **1** may include blocking means on the user's head comprising laces, buckles or tapes **10.**

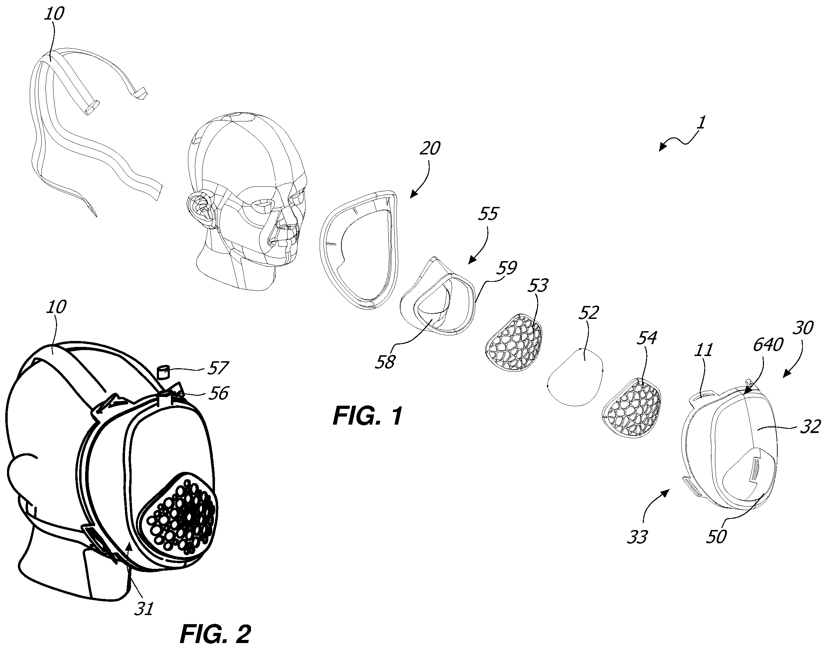

The face-shield device **1** may also include an optically transparent screen **30 ,** and a substantially annular gasket **20** suitably coupled with the screen **30** that mutually cooperate so that once the gasket **20** is located on the patient's face the gasket defines with the latter and with the screen **30** a substantially sealed working chamber **31** which covers eyes, nose and mouth of the patient, as shown in FIGs. 2, 4, 5a, 5b, 5C.

The face-shield device **1** may be substantially rigid. The face-shield device **1** may comprise a screen **30** made of optically transparent thermoplastic polymeric material. The polymeric material may be relatively rigid or semi-rigid and it may be, for example, polymethyl methacrylate (PMMA), PETG, ABS, PST, PE, PC or the like.

The screen **30** may include an external surface **32** and a substantially concave internal surface **33.**

The substantially annular gasket element **20,** may be preferably made of elastomeric material, such as NBR synthetic rubber, silicone or thermoplastic elastomers.

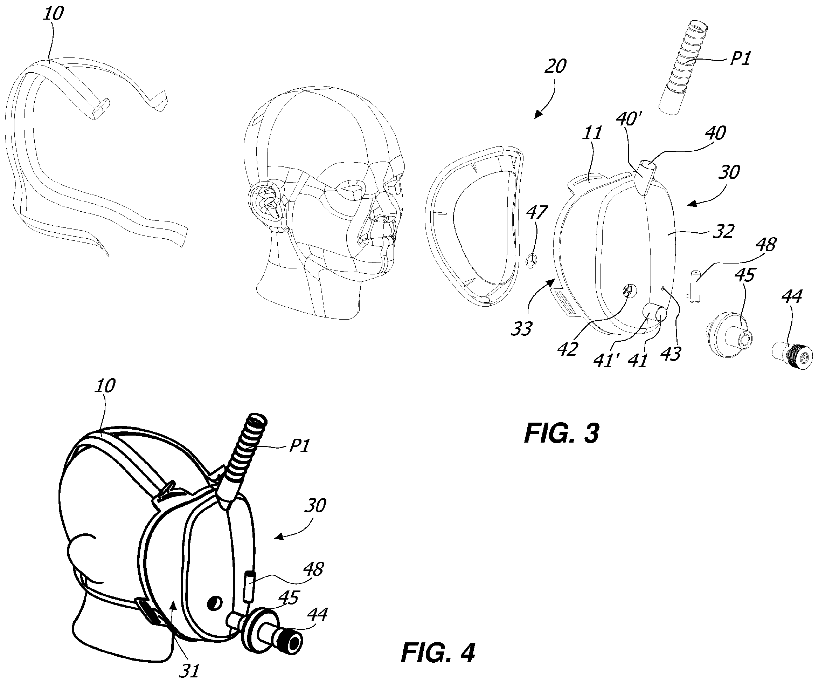

The gasket element **20** may be susceptible to come into contact with the user's face and to define, during use, in cooperation with the internal surface **33** of the screen **30,** the working chamber **31** as specifically shown in FIGs. 1 and 3.

In a first embodiment, shown for example in FIGs. 1 and 3, the elastomeric material element **20** may be removably coupled with the screen **30.**

In another embodiment not shown in the figures, the elastomeric material element **20** may be coupled with the screen **30** by blocking means. For this purpose, the elastomeric material element **20** may include a specific T-slot and centering of the blocking means. In another embodiment not shown in the figures, the elastomeric material element **20** may be monolithic with the screen **30,** for example co-molded with the latter.

The face-shield device **1** may therefore comprise the screen **30,** the elastomeric material element **20** and positioning and blocking means **10** comprising for example tapes, rubbers bands or the like.

Suitably, depending on the configuration of the screen **30** the device **1** may be a personal protection device or a C-PAP device.

Suitably, the screen **30** may include one or more eyelets **11** for the connection by positioning and blocking elements **10** on the head of a user or on a safety helmet not shown in the figures. This will allow the face-shield device **1** to be correctly positioned during use.

According to a first embodiment shown in figures 3 and 4 the device **1** may be a C-PAP device. In such case, the device **1** may comprise means to create an over-pressure in the chamber **31.** The term over-pressure, or positive pressure, indicates a level of pressure that is above the normal atmospheric pressure.

For example, the device **1** may comprise a uniderctional valve **44** of a per se known type in order to maintain an overpressure inside the working chamber **31.**

The device **1** may also comprise a safety membrane anti-suffocation valve **47** in order to avoid depressure in the working chamber **31.**

The device **1** may comprise a pressure gauge **48** connected to the working chamber **31** in order to indicate the assistance medical personnel the existence and the value of the positive pressure existing in the working chamber **31.**

In particular, the screen **30** of the C-PAP medical device may further comprise an opening **40** and the openings **41** and **42** to mutually connect the working chamber **31** with the external environment.

The opening **40** may be defined by a cylindrical element **40'** suitable to be connected with a pipe **P1,** of a per se known type, which in turn may be connected in a per se known manner with adduction means of a working fluid, for example a gas supply line, oxygen and/or air at the desired operating pressure.

The valve **44** may be connected to the cylindrical element **41'** defining the opening **41** or it may be fluidically connected to the latter by a connection and extension pipe **46** and a purification filter **45** for the exhalation air emitted by the user in order to avoid the environment contamination.

The anti-suffocation valve **47** may be located in correspondence or connected with the opening **42.**

The screen **30** may comprise a further opening **48** for the connection of the pressure gauge **48** in order to indicate the medical personnel the existence and the value of the positive pressure existing in the working chamber **31.**

Thanks to what explained above, it is possible to create a PPE device for the mechanical ventilation which does not limit the user's head movements, does not stress the eardrums in case of positive pressure, does not produce a continuous annoying noise due to the passage of gases, and does not exclude the possibility of using the phone or speaking to the personnel. The device **1** may, in fact, be connected to the adduction means of a working fluid through the pipe **P1** and to the unidirectional valve means for the creation of pressure losses **44** directly or through the pipe **46.** When in use, it will be sufficient to place the device **1** on the patient's face, connect the pipe **P1** and turn on the adduction means of the working fluid and read the operating pressure by using the pressure gauge **48.**

The adduction means of the working fluid, the pipe **P1,** the means for the creation of the pressure losses **44,** the pressure gauge **48** and, if present, the pipe **46** may be of a per se known type.

During inhalation, the user will only inhale air or oxygen enriched air coming from the pipe **P1,** while during exhalation the air will pass from the working chamber **31** to the external environment through the uniderctional valve **44,** which creates resistance while passing and therefore creates an over-pressure in the working chamber **31,** and through the filter **45** preventing the exhaled air to contaminate the surrounding environment.

Suitably, the opening **40** is located on the top of the screen **30,** substantially in correspondence with the user's forehead, and the openings **41, 42, 43** are located on the bottom of the screen **30** in correspondence with the user's nose and mouth: in this way there are no connections in correspondence with the user's eyes, and the patient's sight is unobstructed and not impaired by the extremely annoying and depressing sight of pipes.

According to a different embodiment shown in figures 1, 2, 5A, 5B, 5C, the device may be a personal protection device.

In such case, the screen **30** may comprise a first opening **50** to mutually connect the working chamber **31** and the external environment.

Suitably, in order to directly convey the air flow from the outside towards the user's nose/mouth, the working chamber **31** may comprise a substantially frusto-conical element **55** extending from the opening **50** where there is the smallest section of area, to the facial surface of the user where the section of area is bigger and sufficient to include mouth and nose. In such way the component **55** reduces the air dead-volume in the working chamber **31** so that the user does not breathe what he has just exhaled, but retrieves fresh, oxygen-rich air from the outside.

Furthermore, the internal surface of the screen **30** is prevented from fogging.

Suitably the element **55** presents a flexible gasket element **58** near the user's face sealing with the latter and an edging element **59** being able to put pressure on the inner edge, in the working chamber **31,** of the opening **50** in the screen **30.**

Suitably the personal protection device 1 may comprise at least one or more filtering means **52** fixed by blocking means that may have any configuration as long as they perform the function of removably blocking the one or more filtering means **52** in the personal protection device **1**.

According to a preferred embodiment shown in figure 1, the removable blocking means may include one or more transversal elements and/or one or more orthogonal elements to such transversal elements so that they define a sort of grid or they may include perforated sheet-like flat elements **53, 54.**

Suitably, the filtering elements **52** are interposed between the sheet-like elements **53** and **54.**

The filtering elements **52,** of a per se known type, may be made of fibrous textile material, preferably of non-woven fabric.

In a preferred embodiment, the edging element **59** of the substantially frusto-conical element **55** may put pressure on the inner edge of the opening **50** by pressing the same edge against the sheet-like elements **53, 54** which hold the filter element **52** by blocking them.

In a further embodiment of the personal protection device, the screen **30** may have a further opening **56** and a pin protruding element **57** connected or inserted in the opening **56.**

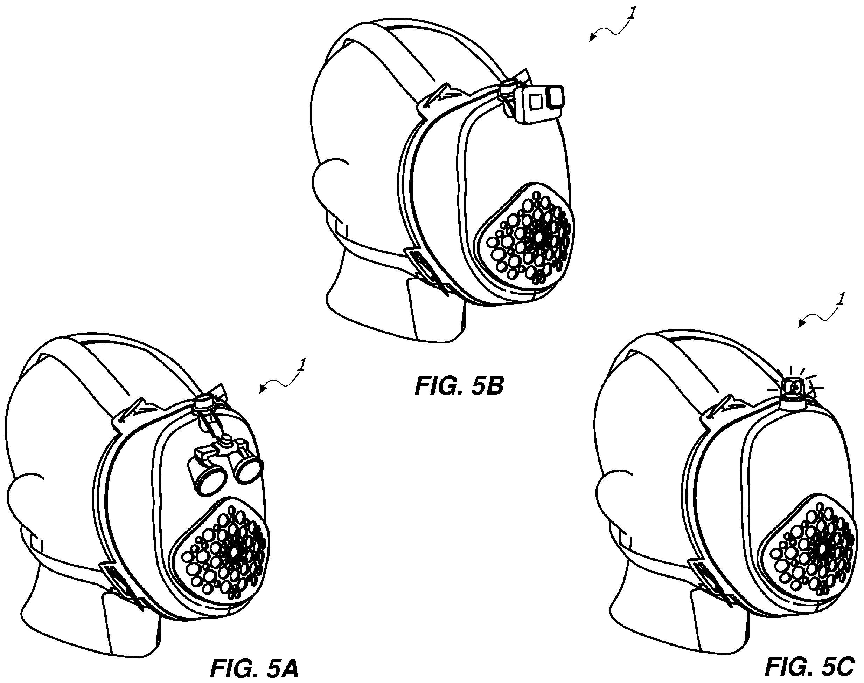

Advantageously, such pin protruding element **57** may be shaped or threaded in order to support accessory elements, such as glasses, cameras, alarms as shown in Figures 5A, 5B, 5C.

For example, during dental surgeries, the personal protection device may also protect the doctor's eyes from contamination and at the same time it may allow the use of lenses as shown in figure 5A or it may record the surgery by using cameras as shown in figure 5B. Both lenses and camera are fixed to the pin element **57.**

As a further example of application, the device **1** may be used as a mask usable for example for concerts, theater, cinema, events, plane, on which, through the pin **57** as shown in figure 5C, lights are applied to indicate whether the mask is properly in use, so that any incorrect use can be immediately detected.

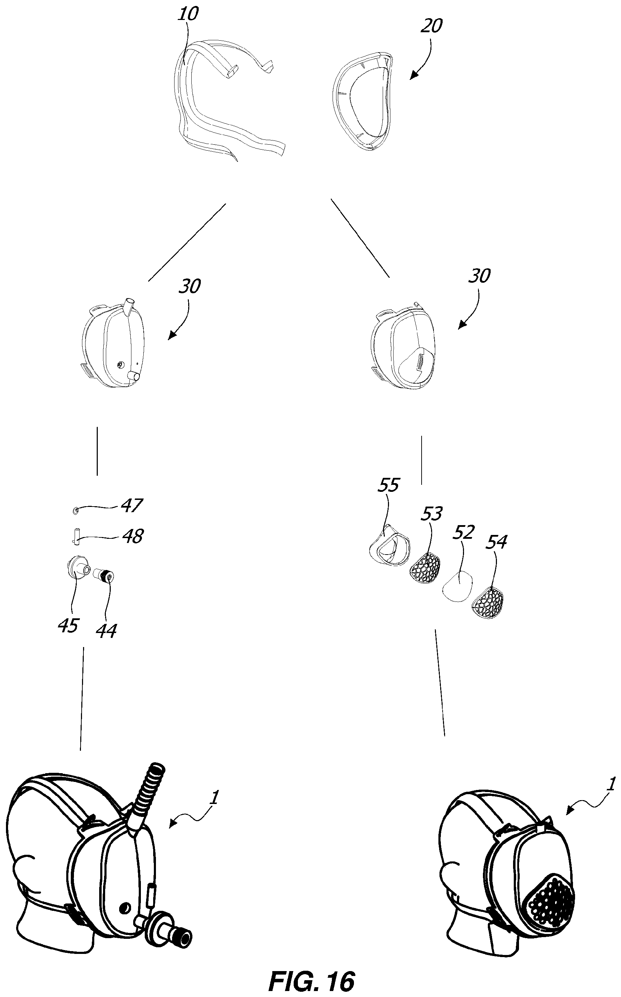

According to the invention, as above described and shown in figure 16, starting from the same common parts **10, 20,** by changing the configuration of the screen **30,** i.e. number and position of the connecting openings of the internal chamber **31** with the outside, a screen **30** suitable for making a personal protection device **1** or a screen suitable for making a C-PAP medical device **1** may be obtained.

The screen **30** may be made of optically transparent polymeric material for example by injection molding.

For the injection molding a single mold with the insertion of suitable inserts in the seats **60', 60"** and **70', 70"** in the die half-mold **60** and correspondingly in the punch half-mold **70** may be used.

More particularly, the seats **60', 70',** preferably having a substantially parallelepipedal planform, may be placed in correspondence of an area **600** of the mold susceptible to make the part of the screen **30** located above the user's eyes. On the other hand, the seats **60", 70",** preferably having a substantially frusto-conical planform, may be placed in correspondence of an area **610** of the mold susceptible to make the part of the screen **30** located in correspondence of the user's nose and mouth.

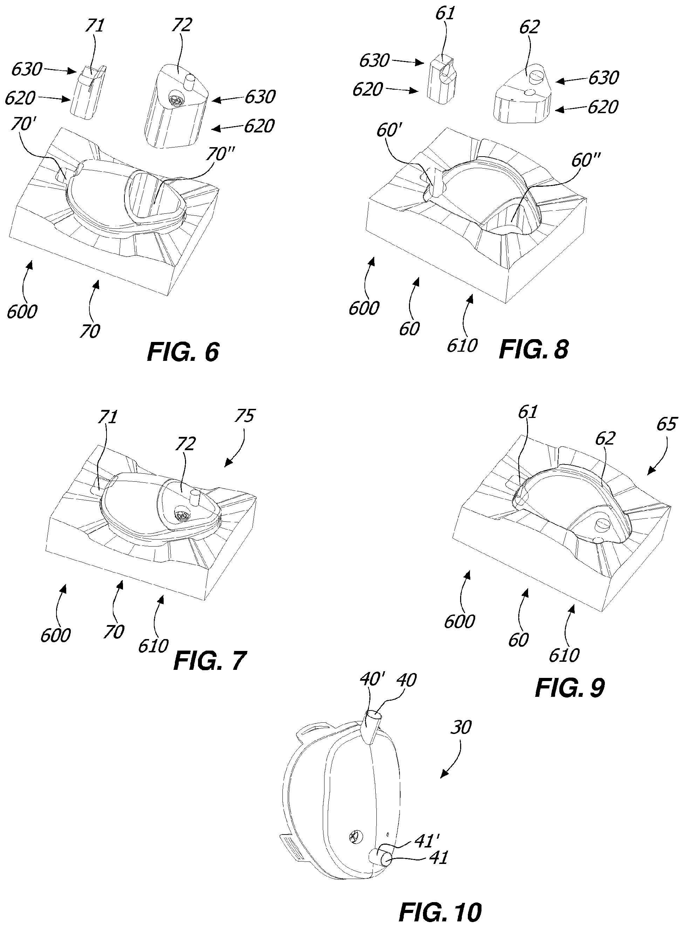

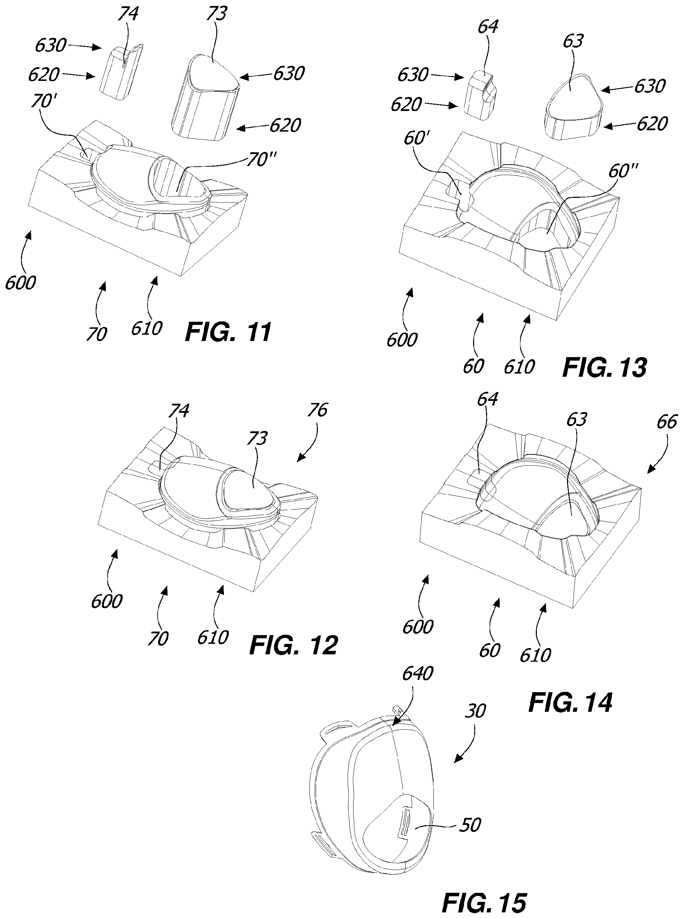

With reference to Figures 8, 9, 13, 14, the substantially concave die half-mold **60,** may comprise a first base portion in whose seats **60', 60"** inserts **61, 62** can be inserted to obtain a screen configured for a C-PAP medical devices, or inserts **63, 64** may be inserted to obtain a screen configured for a personal protection device.

Correspondingly, with reference to figures 6, 7, 11 and 12, the substantially convex punch half-mold **70,** comprises a second base portion in whose seats **70', 70"** inserts **71, 72** can be inserted to obtain a screen configured for a C-PAP device, or inserts **73, 74** can be introduced to obtain a screen configured for a personal protection device.

By inserting the inserts **63, 64** and or the inserts **61, 62** in the first base portion, the matrix **66** for the molding of a screen configured for personal protection device or the **65** matrix for molding a screen configured for a C-PAP device shall be respectively obtained.

Correspondingly, by inserting the inserts **74, 73** or the inserts **71, 72** in the second base portion, the punch **76** for molding a screen configured for a personal protection device or the punch **75** for molding a screen configured for a C-PAP device shall be respectively obtained.

For this purpose, each insert **61, 62; 71, 72; 63, 64; 73, 74** may have an insertion portion **620** in the respective seat **60', 60"; 70', 70"** having a planform substantially correspondent to this latter.

Therefore, the insertion portions **620** of inserts **61** and **64** insertable in the seat **60'** and the insertion portions of inserts **71** and **74** insertable in the seat **70'** may have a substantially parallelepiped planform, while the insertion portions **620** of inserts **62** and **63** insertable in the seat **60"** and insertion portions of inserts **72** and **73** insertable in the seat **70"** may have a substantially frusto-conical planform.

In such way, the inserts **61, 64; 71, 74; 62, 63** and **72, 73** will be interchangeable with each other in their respective seats **60'; 70'; 60"; 70".**

Each of inserts **61, 62; 71, 72; 63, 64; 73, 74** may also have a working portion **630** susceptible to respectively create the through openings **40** and **41** during the molding of the screen **30** for the C-PAP medical devices, and a continuous portion without openings **640** and the opening **50** during the molding of the screen **30** for the personal protection device.

More particularly, the working portion **630** of the inserts pair **61, 71** may cooperate with each other in order to create the through opening **40,** while the working portions **630** of the inserts pair **62, 72** may cooperate with each other in order to create the through opening **41.**

For this purpose, the working portions **630** of the inserts pairs **61, 71** and **62, 72** may be configured in order to create during the molding of the screen **30** for the C-PAP medical device respectively the cylindrical elements **40'** and **41'** leaking from the same screen **30** in order to define the openings **40** and **41.**

On the other hand, the working portions **630** of the inserts pair **64, 74** may cooperate with each other in order to create the continuous portion without openings **640,** while the working portions **630** of the inserts pair **63, 73** may cooperate with each other in order to create the through opening **50.** The continuous portion without openings **640** is indistinguishable from the rest of the screen **30** for the personal protection device, and is an integral part of it.

For this purpose, the working portion **630** of the inserts pairs **64, 74** and **63, 73** may be configured in order to create during the molding of the screen **30** for the personal protection device respectively an area of the same screen **30** coplanar with the surrounding ones to define the continuous portion without openings **640** and the through opening **50.**

In this way, by using one single die half-mold **60** and one single punch half-mold **70,** it is possible to injection-mold the screen **30** for the C-PAP medical device and the screen for the personal protection device simply changing the inserts **61, 64; 71, 74; 62, 63** and **72, 73** in the respective seats **60'; 70'; 60"; 70".**

From what described above, it is clear that the invention reaches the intended purposes.

Thanks to the use of a single-injection mold **60, 70,** indeed, both investment costs and manufacturing costs of the C-PAP medical device and the personal protection device are significantly reduced.

The present invention further allows to create C-PAP medical devices and personal protection devices starting from the same common parts **10, 20** and making the first or second screen **30** simply by replacing the inserts in the single mold.

The invention is susceptible to numerous modifications, all falling within the scope of protection of the attached claims.

## Claims

1. A system for making a first and a second screen **(30)** made of an optically transparent thermoplastic material suitable to cover eyes, nose and mouth of a user, the first screen **(30)** to be used to make a medical face-shield device **(1)** of C-PAP type and comprising at least one first through opening **(40)** connectable with means **(P1)** for the introduction of a pressurized working gas and at least one second opening **(41)** connectable with valve means **(45)** of unidirectional type in order to maintain a positive pressure of said working gas, the second screen **(30)** being usable to create a face-shield device **(1)** of personal protection and comprising at least one third through opening **(50)** operatively connectable with air filtering means **(52);** the system comprising:
- an injection-mold **(60, 70)** for the alternative molding of said first or second screen **(30),** said injection-mold **(60, 70)** comprising a first area **(600)** for obtaining the at least one first through opening **(40)** during the molding of the first screen **(30)** or a continuous portion without openings **(640)** during the molding of the second screen **(30)** and a second area **(610)** for obtaining the at least one second through opening **(41)** during the molding of the first screen **(30)** or the at least one third through opening **(50)** during the molding of the second screen **(30),** said injection-mold **(60, 70)** being formed by a die half-mold **(60)** and a punch half-mold **(70);**
- first inserts **(61, 62; 71, 72)** to be used during the making of said first screen **(30);**
- second inserts **(61, 62; 71, 72)** to be used during the making of said second screen **(30);**
wherein each of said die half-mold **(60)** and punch half-mold **(70)** comprises a first and a second seat **(60', 60"; 70', 70")** for the alternative insertion of said first inserts **(61, 62; 71, 72)** or second inserts **(63, 64; 73, 74);**
wherein said first seats **(60', 70')** of said die half-mold **(60)** and punch half-mold **(70)** are located in correspondence of said first area **(600),** the second seats **(60", 70")** of said die half-mold **(60)** and punch half-mold **(70)** being located in correspondence of said second area **(610);**
wherein said first inserts **(61, 62; 71, 72)** comprise a first pair **(61, 71)** of inserts insertable in said first seats **(60', 70')** cooperating with each other to create said at least one first through opening **(40)** and a second pair **(62, 72)** of inserts insertable in said second seats **(60", 70")** cooperating with each other to create said at least one second through opening **(41);**
wherein said second inserts **(63, 64; 71, 72)** comprise a third pair **(64, 74)** of inserts insertable in said first seats **(60', 70')** cooperating with each other to create said continuous portion without openings **(640)** and a fourth pair **(63, 73)** of inserts insertable in said second seats **(60", 70")** cooperating with each other to create said at least one third through opening **(41);**
so that the first and the second screen **(30)** are injection-moldable by one single mold made of said injection-mold **(60, 70)** depending on the insertion in the first and second seats **(60', 60"; 70', 70")** of the first inserts **(61, 62; 71, 72)** or second inserts **(63, 64; 73, 74).**

2. System according to claim 1, wherein said first and second seats **(60', 60"; 70', 70")** have respective predetermined planforms each of said first inserts **(61, 62; 71, 72)** and second inserts **(63, 64; 73, 74)** having a first insert portion that can be inserted in the respective first or second seat **(60', 60"; 70', 70")** having a planform substantially corresponding to that of the latter and a second working portion susceptible to create respectively said at least one first through opening **(40),** said at least one second through opening(**41**), said continuous portion without openings **(640)** and said at least one third through opening **(50).**

3. System according to the preceding claim, wherein said first seats **(60', 70')** have a substantially parallelepiped planform, said second seats **(60", 70")** having a substantially frusto-conical planform.

4. System according to claims 2 or 3, wherein the second portions of said first pair **(61, 71)** of inserts are mutually configured in order to create during the molding of the first screen **(30)** a first cylindrical **(40')** element leaking out from the latter in order to define the at least one first through opening **(40).**

5. System according to claims 2, 3 or 4, wherein the second portions of said second pair **(62, 72)** of inserts are mutually configured in order to create during the molding of the first screen **(30)** a second cylindrical element **(41')** leaking from the latter in order to define the at least one second through opening **(41).**

6. System according to claims 2, 3, 4 or 5, wherein the second portions of said third pair **(64, 74)** of inserts are mutually configured in order to create during the molding of the second screen **(30)** an area of the latter which is coplanar to the surrounding ones in order to define said continuous portion without openings **(640).**

7. System according to claims 2, 3, 4, 5 or 6, wherein the second portions of said fourth pair **(63, 73)** of inserts are mutually configured in order to create during the molding of the second screen **(30)** a through opening in the latter defining the at least one third through opening **(50).**

8. System according to one or more of the preceding claims, wherein said first area of said injection-mold **60, 70)** is susceptible to create the part of said first and second screen **(30)** located above the user's eyes, said second area of said injection-mold **(60, 70)** being susceptible to create the part of said first and second screen **(30)** located in correspondence with the user's nose and mouth.

9. System according to one or more of the preceding claims, further comprising
- means **(10)** for fixing the face-shield device to the user,
- a substantially annular gasket element **(20)** susceptible to come into contact with the user's face;
wherein said fixing means **(10)** and the substantially annular gasket element **(20)** are mutually connectable with both said first screen **(30)** and said second screen **(30).**

10. A method for alternatively making a first and a second screen **(30)** made of an optically transparent thermoplastic material by the system in accordance with the preceding claims, the method including the steps of:
- providing said one single injection-mold made of said mold **(60, 70);**
- providing said first inserts **(61, 62; 71, 72)** and said second inserts **(63, 64; 73, 74);**
- alternative insertion in said first and second seats **(60', 60''; 70', 70")** of said first inserts **(63, 64; 73, 74)** or said second inserts **(61, 62; 71, 72);**
- injection in said mold **(60, 70)** of said optically transparent thermoplastic material;
in order to mold the first or second screen **(30)** by said single injection-mold **(60, 70)** depending on the insertion in the first and second seats **(60', 60"; 70', 70")** of the first inserts **(61, 62; 71, 72)** or of the second inserts **(63, 64; 73, 74).**

11. A method for making a face-shield device **(1)** including the steps of:
- providing means **(10)** for fixing the face-shield device to a user;
- providing a substantially annular gasket element **(20)** susceptible to come into contact with the user's face;
- providing a first or a second screen **(30)** being optically transparent;
- mutual connection of said fixing means **(10),** said substantially annular gasket element **(20)** and of said first or second screen **(30);**
wherein said step of providing said first or second screen **(30)** includes the step of making the latter by the method according to the preceding claim.

12. Method according to claim 11, wherein said first screen **(30)** is made by the method according to claim 10 wherein in the first and second seats **(60', 60"; 70', 70")** of said single injection-mold **(60, 70)** said first inserts **(63, 64; 73, 74)** are inserted, the method further including the assembly step of unidirectional valve means **(45)** in order to maintain a positive pressure of a working gas in correspondence with said at least one second opening **(41),** so that said face-shield device **(1)** is a C-PAP medical device.

13. Method according to claim 11, wherein said first or second screen **(30)** is made by the method according to claim 10 wherein in the first and second seats **(60', 60"; 70', 70")** of said one single injection-mold **(60, 70)** said second inserts **(61, 62; 71, 72),** are inserted, the method further including the assembly step of the means **(52)** for filtering the air in correspondence of said at least one third opening **(50),** so that said face-shield device **(1)** is a personal protection device.

## Patentansprüche

1. System zur Herstellung eines ersten und zweiten Schirms (30), die aus einem optisch transparenten thermoplastischen Material gefertigt sind, das dazu geeignet ist, Augen, Nase und Mund eines Benutzers zu bedecken, wobei der erste Schirm (30) zur Herstellung eines medizinischen Gesichtsschutzes (1) des Typs C-PAP vorgesehen ist, der wenigstens eine erste Durchtrittsöffnung (40) umfasst, die mit Mitteln (P1) zur Einbringung eines unter Druck befindlichen Arbeitsgases verbindbar ist und die wenigstens eine zweite Öffnung (41) umfasst, die mit unidirektionalen Ventilmitteln (45) verbindbar ist, um einen positiven Druck des Arbeitsgases aufrechtzuerhalten, und der zweite Schirm (30), zur Herstellung eines Gesichtsschutzes (1) zum persönlichen Schutz verwendbar ist und eine dritte Durchtrittsöffnung (50) umfasst, die mit Luftfiltermitteln (52) in Wirkverbindung gebracht werden kann;
das System umfassend:
- ein Spritzgießwerkzeug (60, 70) für das abwechselnde Formen des ersten oder des zweiten Schirms (30), wobei das Spritzgießwerkzeug (60, 70) einen ersten Bereich (600) zum Erhalten der wenigstens einen ersten Durchführungsöffnung (40) während des Formens des ersten Schirms (30) oder einen ununterbrochenen Bereich ohne Öffnungen (640) während der Herstellung des zweiten Schirms (30) und einen zweiten Bereich (610) zum Erhalten der wenigstens einen zweiten Durchführungsöffnung (41) während der Herstellung des ersten Schirms (30) oder der dritten Durchführungsöffnung (50) während der Herstellung des zweiten Schirms umfasst, wobei das Spritzgießwerkzeug (60, 70) durch eine Matrizen-Halbform (60) und eine Stempel-Halbform (70) gebildet wird;
- erste Einsätze (61, 62; 71, 72) zur Verwendung während der Herstellung des ersten Schirms (30);
- zweite Einsätze (61, 62; 71, 72) zur Verwendung während der Herstellung des zweiten Schirms (30);
wobei jede der Matrizen-Halbform (60) und der Stempel-Halbform (70) erste und zweite Sitze (60', 60"; 70', 70'') zum abwechselnden Einsetzen der ersten Einsätze (61, 62; 71, 72) oder der zweiten Einsätze (63, 64; 73, 74) umfasst;
wobei die ersten Sitze (60',70') der Matrizen-Halbform (60) und der Stempel-Halbform (70) entsprechend dem ersten Bereich (600) angeordnet sind, die zweiten Sitze (60'', 70'') der Matrizen-Halbform (60) und der Stempel-Halbform (70) entsprechend dem zweiten Bereich (610) angeordnet sind;
wobei die ersten Einsätze (61, 62; 71, 72) ein erstes Paar (61, 71) von Einsätzen umfassen, die in die ersten Sitze (60', 70') einsetzbar sind, die miteinander zusammenwirken, um die wenigstens eine erste Durchführungsöffnung (40) zu erzeugen und ein zweites Paar (62, 72 ) von Einsätzen umfassen, die in die zweiten Sitze (60'', 70'') einsetzbar sind, die miteinander zusammenwirken, um die wenigstens eine zweite Durchführungsöffnung (41) zu bilden;
wobei die zweiten Einsätze (63, 64; 71, 72) ein drittes Paar (64, 74) von Einsätzen umfasst, die in die ersten Sitze (60', 70') einsetzbar sind und die miteinander zusammenwirken, um den kontinuierlichen Bereich ohne Öffnungen (640) zu bilden und ein viertes Paar (63, 73) von Einsätzen umfassen, die in die zweiten Sitze (60'', 70'') einsetzbar sind und die miteinander zusammenwirken, um die wenigstens eine dritte Durchführungsöffnung (41) zu bilden;
so, dass der erste und zweite Schirm (30) mit einem einzigen Spitzgießwerkzeug (60,70) spritzgegossen werden können, das in Abhängigkeit von dem Einsetzen der ersten und zweiten Sitze (60', 60''; 70', 70'') der ersten Einsätze (61, 62; 71, 72) oder der zweiten Einsätze (63, 64; 73, 74) gebildet wird.

2. System nach Anspruch 1, bei welchem die ersten und zweiten Sitze (60', 60"; 70', 70'') jeweils vordefinierte Grundrisse aufweisen, wobei jeder der ersten Einsätze (61, 62; 71, 72) und der zweiten Einsätze (63, 64; 73, 74) einen ersten Einsatzteil aufweisen, der in den jeweiligen ersten oder zweiten Sitz (60', 60"; 70', 70")mit einem Grundriss, der im Wesentlichen dem Letzteren entspricht, einsetzbar ist und ein zweites Arbeitsteil aufweist, das dazu ausgebildet ist, jeweils die wenigstens eine erste Durchführungsöffnung (40), die wenigstens eine zweite Durchführungsöffnung (41), den ununterbrochenen Bereich ohne Öffnungen (640) und die wenigstens eine dritte Durchführung (50) zu bilden.

3. System nach dem vorhergehenden Anspruch, bei welchem die ersten Sitze (60' 70') einen im wesentlichen quaderförmigen Grundriss aufweisen und die zweiten Sitze (60'', 70'') einen im wesentlichen kegelstumpfförmigen Grundriss aufweisen.

4. System nach einem der Ansprüche 2 oder 3, bei welchem die zweiten Bereiche des ersten Paars (61, 71) von Einsätzen gegenseitig so ausgebildet sind, dass sie während des Formens des ersten Schirms (30) ein erstes zylindrisches Element (40' erzeugen, welches aus dem Letzteren ausläuft, um die wenigstens eine erste Durchführungsöffnung (40) zu erzeugen.

5. System nach den Ansprüchen 2, 3 oder 4, bei welchem die zweiten Bereiche des zweiten Paars (62, 72) der Einsätze gegenseitig so ausgebildet sind, dass sie während des Formens des ersten Schirms (30) ein zweites zylindrisches Element (41') erzeugen, das von Letzteren ausläuft, um die wenigstens eine zweite Durchführungsöffnung (41) zu definieren.

6. System nach den Ansprüchen 2, 3, 4 oder 5, bei welchem die zweiten Bereiche des dritten Paars (64, 74) von Einsätzen gegenseitig so ausgebildet sind, dass sie während des Formens des zweiten Schirms (30) einen Bereich des Letzteren erzeugen, der coplanar mit den umgebenden Bereichen ist, um den ununterbrochenen Bereich ohne Öffnungen (640) zu definieren.

7. System nach den Ansprüchen 2, 3, 4, 5 oder 6, bei welchem die zweiten Bereiche des vierten Paars (63, 73) der Einsätze gegenseitig so ausgebildet sind, dass sie während des Formens des zweiten Schirms (30) eine Durchführungsöffnung in letzterem erzeugen, die die wenigstens eine dritte Durchführungsöffnung (50) definiert.

8. System nach einem oder mehreren der vorhergehenden Ansprüche, bei welchem der erste Bereich des Spritzgießwerkzeugs (60, 70) dazu ausgebildet ist, den Teil des ersten und zweiten Schirms (30) zu erzeugen, der oberhalb des Auges des Benutzers angeordnet ist, wobei der zweite Bereich des Spritzgießwerkzeugs (60, 70) dazu ausgebildet ist, den Teil des ersten und zweiten Schirms (30) zu erzeugen, der entsprechend Nase und Mund des Benutzers angeordnet ist.

9. System nach einem oder mehreren der vorhergehenden Ansprüche, weiterhin umfassend
- Mittel (10) zur Befestigung des Gesichtsschutzes an dem Benutzer;
- ein im wesentlichen ringförmiges Dichtelement (20), das dazu ausgebildet ist, in Eingriff mit dem Gesicht des Benutzers zu gelangen;
wobei die Befestigungsmittel (10) und das im wesentlichen ringförmige Dichtungselement (20) sowohl mit dem ersten Schirm (30) als auch mit dem zweiten Schirm (30) verbindbar sind.

10. Verfahren zur abwechselnden Herstellung erster und zweiter Schirme (30) aus einem optisch transparenten thermoplastischen Material mittels des Systems gemäß der vorstehenden Ansprüche, wobei das Verfahren folgende Schritte umfasst:
- Bereitstellen eines einzigen Spritzgießwerkzeugs, das aus den Spritzgießformen (60, 70) erzeugt wird;
- Bereitstellen der ersten Einsätze (61, 62; 71, 72) und der zweiten Einsätze (63, 64; 73, 74);
- abwechselndes Einsetzen der ersten Einsätze (63, 64; 73, 74) oder der zweiten Einsätze (61, 62; 71, 72) in die ersten und zweiten Sitze (60', 60"; 70', 70");
- Einspritzen des optisch transparenten thermoplastischen Materials in das Spritzgießwerkzeug (60, 70), um den ersten oder zweiten Schirm (30) durch das einzige Spritzgießwerkzeug (60, 70) in Abhängigkeit des Einsetzens der ersten Einsätze (61, 62; 71, 72) oder der zweiten Einsätze (63, 64; 73, 74) in die ersten und zweiten Sitze (60', 60"; 70', 70") zu formen.

11. Verfahren zur Herstellung eines Gesichtsschutzes (1) umfassend folgende Schritte:
- Bereitstellen von Mitteln (10) zur Befestigung des Gesichtsschutzes an einem Benutzer;
- Bereitstellen eines im wesentlichen ringförmigen Dichtelements (20), das dazu ausgebildet ist, in Eingriff mit dem Gesicht des Benutzers zu gelangen;
- Bereitstellen eines ersten oder eines zweiten Schirms (30), der optisch transparent ist;
- gegenseitiges Verbinden der Befestigungsmittel (10), des im wesentlichen ringförmigen Dichtungselements (20) und des ersten oder zweiten Schirms (30);
wobei der Schritt der Bereitstellung des ersten oder zweiten Schirms (30) den Schritt der Herstellung des Letzteren durch das Verfahren gemäß dem vorhergehenden Anspruch umfasst.

12. Verfahren nach Anspruch 11, bei welchem der erste Schirm (30) nach dem Verfahren gemäß Anspruch 10 hergestellt wird, wobei in die ersten und zweiten Sitze (60', 60''; 70', 70'') des Spritzgießwerkzeugs (60, 70) die ersten Einsätze (63, 64; 73, 74) eingesetzt werden, wobei das Verfahren weiterhin den Montageschritt der unidirektionalen Ventilmittel (45) umfasst, um einen positiven Druck eines Arbeitsgases in Kommunikation mit der wenigstens einen Durchführungsöffnung (41) aufrechtzuerhalten, sodass der Gesichtsschutz (1) eine C-PAP medizinische Vorrichtung ist.

13. Verfahren nach Anspruch 11, bei welchem der erste oder zweite Schirm (30) durch das Verfahren gemäß Anspruch 10 hergestellt wird, wobei in die ersten und zweiten Sitze (60', 60"; 70', 70'') des einzigen Spritzgießwerkzeugs (60, 70) die zweiten Einsätze (61, 62; 71, 72) eingesetzt werden, wobei das Verfahren weiterhin den Montageschritt der Mittel (52) zur Filterung der Luft in Kommunikation mit der wenigstens einen dritten Öffnung (50) umfasst, sodass der Gesichtsschutz (1) eine persönliche Schutzvorrichtung ist.

## Revendications

1. Système de fabrication d'un premier et d'un deuxième écran **(30)** en matériau thermoplastique optiquement transparent, aptes à couvrir les yeux, le nez et la bouche d'un utilisateur, le premier écran **(30)** est destiné à la fabrication d'un dispositif médical de protection faciale **(1)** de type C-PAP et comprenant au moins une première ouverture traversante **(40)** connectable à des moyens **(P1)** d'introduction d'un gaz de travail sous pression et au moins une deuxième ouverture **(41)** connectable à des moyens de soupape **(45)** de type unidirectionnelle afin de maintenir une pression positive dudit gaz de travail, le deuxième écran **(30)** pouvant être utilisé pour la fabrication d'un dispositif de protection faciale **(1)** de protection personnelle et comprenant au moins une troisième ouverture traversante **(50)** connectable fonctionnellement à des moyens de filtration d'air **(52);**
le système comprenant:
- un moule d'injection **(60, 70)** pour le moulage alternatif dudit premier ou deuxième écran **(30),** ledit moule d'injection **(60, 70)** comprenant une première zone **(600)** pour obtenir ladite au moins une première ouverture traversante **(40)** lors du moulage du premier écran **(30)** ou une partie continue sans ouvertures **(640)** lors du moulage du deuxième écran **(30)** et une deuxième zone **(610)** pour obtenir ladite au moins une deuxième ouverture traversante **(41)** lors du moulage du premier écran **(30)** ou ladite au moins une troisième ouverture traversante **(50)** lors du moulage du deuxième écran **(30),** ledit moule d'injection **(60, 70)** étant formé par un demi-moule matrice **(60)** et un demi-moule poinçon **(70);**
- des premiers inserts **(61, 62; 71, 72)** destinés à être utilisés lors de la fabrication dudit premier écran **(30);**
- des deuxièmes inserts **(61, 62; 71, 72)** destinés à être utilisés lors de la fabrication
dudit deuxième écran **(30);**
dans lequel chacun desdits demi-moule matrice **(60)** et demi-moule poinçon **(70)** comprend
un premier et un deuxième logement **(60', 60"; 70', 70")** pour l'insertion alternative desdits premiers inserts **(61, 62; 71, 72)** ou deuxièmes inserts **(63, 64; 73, 74);**
dans lequel lesdits premiers logements **(60', 70')** desdits demi-moule matrice **(60)** et demi-moule poinçon **(70)** sont situés en correspondance de ladite première zone **(600),** les deuxièmes logements **(60", 70")** desdits demi-moule matrice **(60)** et demi-moule poinçon **(70)** étant situés en correspondance de ladite deuxième zone **(610);**
dans lequel lesdits premiers inserts **(61, 62; 71, 72)** comprennent une première paire **(61, 71)** d'inserts insérables dans lesdits premiers logements **(60', 70')** coopérant l'un avec l'autre pour créer ladite au moins une première ouverture traversante **(40)** et une deuxième paire **(62, 72)** d'inserts insérables dans lesdits deuxièmes logements **(60", 70")** coopérant l'une avec l'autre pour créer ladite au moins une deuxième ouverture traversante **(41);**
dans lequel lesdits deuxièmes inserts **(63, 64; 71, 72)** comprennent une troisième paire
**(64, 74)** d'inserts insérables dans lesdits premiers logements **(60', 70')** coopérant l'un avec l'autre pour créer ladite partie continue sans ouvertures **(640)** et une quatrième paire **(63, 73)** d'inserts insérables dans lesdits deuxièmes logements **(60", 70")** coopérant l'un avec l'autre pour créer ladite au moins une troisième ouverture **(41);**
de sorte que le premier et le deuxième écran **(30)** sont moulables par injection par un seul moule réalisé à partir dudit moule d'injection **(60, 70)** en fonction de l'insertion dans les premiers et deuxièmes logements **(60', 60"; 70', 70")** des premiers inserts **(61, 62; 71, 72)** ou des deuxièmes inserts **(63, 64; 73, 74).**

2. Système selon la revendication 1, dans lequel lesdits premiers et deuxièmes logements **(60', 60"; 70', 70")** ont des formes en plan respectives prédéterminées, chacun desdites premiers inserts **(61, 62; 71, 72)** et deuxièmes inserts **(63, 64; 73, 74)** ayant une première partie d'insert qui peut être insérée dans le premier ou le deuxième logement respectif **(60', 60"; 70', 70")** ayant une forme en plan correspondant sensiblement à celle de ce dernier et une deuxième partie de travail susceptible de créer respectivement ladite au moins une première ouverture traversante **(40),** ladite au moins une deuxième ouverture traversante **(41),** ladite partie continue sans ouvertures **(640)** et ladite au moins une troisième ouverture traversante **(50).**

3. Système selon la revendication précédente, dans lequel lesdits premiers logements
**(60', 70')** ont une forme en plan sensiblement parallélépipédique, lesdits deuxièmes logements **(60", 70")** ayant une forme en plan sensiblement tronconique.

4. Système selon les revendications 2 ou 3, dans lequel les deuxièmes parties de ladite
première paire **(61, 71)** d'inserts sont mutuellement configurées afin de créer, lors du moulage du premier écran **(30),** un premier élément cylindrique **(40')** s'échappant hors de ce dernier afin de définir l'au moins une première ouverture traversante **(40).**

5. Système selon les revendications 2, 3 ou 4, dans lequel les deuxièmes parties de ladite deuxième paire **(62, 72)** d'inserts sont mutuellement configurées afin de créer, lors du moulage du premier écran **(30),** un deuxième élément cylindrique **(41')** s'échappant hors de ce dernier afin de définir l'au moins une deuxième ouverture traversante **(41).**

6. Système selon les revendications 2, 3, 4 ou 5, dans lequel les deuxièmes parties de
ladite troisième paire **(64, 74)** d'inserts sont mutuellement configurées afin de créer, lors du moulage du deuxième écran **(30),** une zone de ce dernier qui est coplanaire à celles environnantes afin de définir ladite partie continue sans ouvertures **(640).**

7. Système selon les revendications 2, 3, 4, 5 ou 6, dans lequel les deuxièmes parties de ladite quatrième paire **(63, 73)** d'inserts sont mutuellement configurées afin de créer, lors du moulage du deuxième écran **(30),** une ouverture traversante dans ce dernier définissant l'au moins une troisième ouverture traversante **(50).**

8. Système selon l'une ou plusieurs des revendications précédentes, dans lequel ladite
première zone dudit moule d'injection **(60, 70)** est susceptible de créer la partie desdits premier et deuxième écran **(30)** située au-dessus des yeux de l'utilisateur, ladite deuxième zone dudit moule d'injection **(60, 70)** étant susceptible de créer la partie desdits premier et deuxième écran **(30)** située en correspondance avec le nez et la bouche de l'utilisateur.

9. Système selon une ou plusieurs des revendications précédentes, comprenant en outre
- des moyens **(10)** de fixation du dispositif de protection facial à l'utilisateur,
- un élément d'étanchéité sensiblement annulaire **(20)** susceptible d'entrer en contact
avec le visage de l'utilisateur;
dans lequel lesdits moyens de fixation **(10)** et l'élément d'étanchéité sensiblement annulaire **(20)** sont mutuellement connectables à la fois audit premier écran **(30)** et audit deuxième écran **(30).**

10. Procédé de fabrication alternative d'un premier et d'un deuxième écran **(30)** en matériau thermoplastique optiquement transparent par le système selon les revendications précédentes , le procédé comprenant les étapes suivantes:
- la fourniture dudit moule d'injection unique constitué dudit moule **(60, 70);**
- la fourniture desdits premiers inserts **(61, 62; 71, 72)** et desdits deuxièmes inserts **(63, 64; 73, 74);**
- l'insertion alternative dans lesdits premiers et deuxièmes logements **(60', 60"; 70', 70")** desdits premiers inserts **(63, 64; 73, 74)** ou desdits deuxièmes inserts **(61, 62; 71, 72);**
- l'injection dans ledit moule **(60, 70)** dudit matériau thermoplastique optiquement
transparent;
afin de mouler le premier ou le deuxième écran **(30)** par ledit moule d'injection
unique **(60, 70)** en fonction de l'insertion dans les premiers et les deuxièmes logements **(60', 60"; 70', 70")** des premiers inserts **(61, 62; 71, 72)** ou des deuxièmes inserts **(63, 64; 73, 74).**

11. Procédé de fabrication d'un dispositif de protection facial **(1)** comprenant les étapes
suivantes:
- la fourniture de moyens **(10)** de fixation du dispositif de protection faciale à un utilisateur;
- la fourniture d'un élément d'étanchéité sensiblement annulaire **(20)** susceptible d'entrer en contact avec le visage de l'utilisateur;
- la fourniture d'un premier ou d'un deuxième écran **(30)** étant optiquement transparent;
- la connexion mutuelle desdits moyens de fixation **(10),** dudit élément d'étanchéité sensiblement annulaire **(20)** et desdits premier ou deuxième écran **(30);**
dans lequel ladite étape de fourniture desdits premier ou deuxième écran **(30)** comprend l'étape de fabrication de ce dernier par le procédé selon la revendication précédente.

12. Procédé selon la revendication 11, dans lequel ledit premier écran **(30)** est fabriqué
par le procédé selon la revendication 10, dans lequel dans les premiers et deuxièmes logements **(60', 60"; 70', 70")** dudit moule d'injection unique **(60, 70),** lesdits premiers inserts **(63, 64; 73, 74)** sont insérés, le procédé comprenant en outre l'étape d'assemblage des moyens de soupape unidirectionnelle **(45)** afin de maintenir une pression positive d'un gaz de travail en correspondance avec ladite au moins une deuxième ouverture **(41),** de sorte que ledit dispositif de protection faciale **(1)** est un dispositif médical C-PAP.

13. Procédé selon la revendication 11, dans lequel ledit premier ou deuxième écran **(30)**
est fabriqué par le procédé selon la revendication 10, dans lequel dans les premiers et deuxièmes logements **(60', 60"; 70', 70")** dudit moule d'injection unique **(60, 70),** lesdits deuxièmes inserts **(61, 62; 71, 72),** sont insérés, le procédé comprenant en outre l'étape d'assemblage des moyens **(52)** de filtration de l'air en correspondance de ladite au moins une troisième ouverture **(50),** de sorte que ledit dispositif de protection faciale **(1)** est un dispositif de protection personnelle.
